# EUROPEAN PATENT APPLICATION

(11) **EP 2 615 110 A1**
(43) Date of publication of application: **17.07.2013**
(21) Application number: 12193633.0
(22) Date of filing: 21.11.2012
(51) Int. Cl.: C07K 14/705, G01N 33/50, G01N 33/574

(54) **Biomarker for diagnosing cancer and method of isolating cancer cell using the same**

(30) Priority: 10.01.2012 KR 20120003081
(71) Applicant: Samsung Electronics Co., Ltd, Gyeonggi-do 443-742 (KR); Ajou University Industry Cooperation Foundation, Suwon-si, Gyeonggi-do 443-380 (KR)
(72) Inventor: Kim, Yeon-jeong, Yongin-si, Gyeonggi-do (KR); Kim, You-sun, Suwon-si, Gyeonggi-do (KR); Lee, Jeong-gun, Yongin-si, Gyeonggi-do (KR); Baek, Sang-hyun, Yongin-si, Gyeonggi-do (KR); Oh, Jin-mi, Yongin-si, Gyeonggi-do (KR); Jeong, Hyo-young, Yongin-si, Gyeonggi-do (KR)
(74) Representative: Grünecker, Kinkeldey, Stockmair & Schwanhäusser

(57) **Abstract**

A biomarker for detecting a cancer stem cell or a circulating cancer cell that has undergone epithelial-mesenchymal transition, a kit for detecting a cancer stem cell or a circulating cancer cell that has undergone epithelial-mesenchymal transition, the kit including an antibody against the biomarker, a method of diagnosing cancer by using the kit, and a method of isolating a cancer stem cell or a circulating cancer cell that has undergone epithelial-mesenchymal transition. By using the biomarker for cancer diagnosis and the kit including an antibody against the biomarker, a cancer stem cell or a circulating cancer cell that has undergone epithelial-mesenchymal transition is efficiently detected and isolated.

## Description

### BACKGROUND

### 1. Field

The present disclosure relates to biomarkers for detecting cancer stem cells or circulating cancer cells that have undergone epithelial-mesenchymal transition, kits for detecting cancer stem cells or circulating cancer cells that have undergone epithelial-mesenchymal transition, the kits including the biomarkers, methods of detecting Caveolin-1, in particular for diagnosing cancer, and methods of isolating cancer stem cells or circulating cancer cells that have undergone epithelial-mesenchymal transition.

### 2. Description of the Related Art

Tumor metastasis, which occurs in patients with solid cancer, is a process that involves the release of single tumor cells and migration of these cells to other parts of the body via blood vessels, and is a major cause of death due to cancer. Currently, biopsy, which is a process whereby a portion of a tissue at an early stage of metastasis is taken out and analyzed, is generally used to diagnose cancer. However, this method is disadvantageous in that it is not easy to determine an accurate biopsy site. On the other hand, liquid biopsy that has recently drawn attention is a method whereby only a blood sample is simply collected and a tumor cell in the blood is detected. This method is advantageous in confirming the progress and treatment of cancer and in detecting and diagnosing cancer at an early stage.

As test methods that rapidly confirm the treatment results of patients with metastatic cancer and sensitively reflect the efficacy of a medicine are strongly required, the detection of circulating tumor cells (CTCs) present in the bloodstream of patients with cancer through tumor invasion and disseminated tumor cells (DTCs) present in other organs of a patient but not found clinically has gained attention. CTCs are rare tumor cells present in blood and thus circulating in the body and play a critical role in metastasis of tumors. Accordingly, the detection of tumor cells in blood remains an important task in the diagnosis and treatment of cancer.

Currently, as an anti-cancer treatment method, anti-cancer drugs are administered to most patients in all cases without confirming the presence or absence of CTCs or DTCs. Thus, selective administration of anti-cancer drugs according to the presence or absence of CTCs by detecting and analyzing CTCs or improvement in the efficacy of anti-cancer drugs through customized administration according to the molecular properties of CTCs is needed.

CTCs are known to be involved in the metastasis and recurrence of cancer. In particular, it has been suggested that the CTCs are likely to contain cancer stem cells, which is one of the most important subjects of recent cancer research. Therefore, a new possibility of predicting prognosis of cancer, which cannot be obtained via the existing biopsy, by analyzing CTCs and a possibility of developing patient-customized therapies based thereon are expected.

However, CTCs are contained in blood in a very small amount, and thus, it is very difficult to detect CTCs and count the number thereof. Therefore, there is still a need to develop a diagnosis method with high sensitivity that can detect CTCs, cancer cells or cancer stem cells present in the body of patients, a method of efficiently isolating CTCs contained in a biological sample, and an apparatus related thereto.

### SUMMARY

Provided are biomarkers for detecting cancer stem cells or circulating cancer cells that have undergone epithelial-mesenchymal transition, the biomarkers comprising Caveolin-1.

Provided are kits for detecting cancer stem cells or circulating cancer cells that have undergone epithelial-mesenchymal transition, the kits including an antibody specifically binding to Caveolin-1 or a fragment thereof or an antigen binding fragment thereof.

Provided are methods of detecting Caveolin-1 in a biological sample, in order to provide information needed for diagnosis of cancer.

Provided are methods of isolating cancer stem cells or circulating cancer cells that have undergone epithelial-mesenchymal transition by using Caveolin-1 as a biomarker.

### BRIEF DESCRIPTION OF THE DRAWINGS

These and/or other aspects will become apparent and more readily appreciated from the following description of the embodiments, taken in conjunction with the accompanying drawings in which:

FIG. 1 illustrates western blotting results showing an expression level of Caveolin-1 in 9 types of cancer cell lines, according to an embodiment of the present invention;

FIGS. 2A through 2C are images showing immunocytochemistry and western blotting results confirming that epithelial-mesenchymal transition was induced in MCF7 cell lines, in which sphere denotes epithelial-mesenchymal transition-induced cells, according to embodiments of the present invention;

FIG. 3 illustrates images showing results of comparing the binding affinity of beads with a Caveolin-1 antibody bound thereto to a MCF-7 cell that has undergone epithelial-mesenchymal transition with the binding affinity of the beads to a normal cancer cell, according to an embodiment of the present invention; and

FIG. 4 illustrates images showing western blotting results that confirm expression levels of Caveolin-1, Snail, ALDH1, CD133, and CD44 in epithelial-mesenchymal transition-induced MCF7 cells, according to an embodiment of the present invention.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description.

According to an embodiment of the present invention, there is provided a biomarker for detecting a cancer stem cell or a circulating cancer cell that has undergone epithelial-mesenchymal transition, the biomarker including Caveolin-1 having a higher expression level in a cancer cell than a normal cell.

Caveolin-1 is a protein encoded by CAV1, and is known to be a major protein present in caveolae which are invaginations of the cell membrane found in most types of human cells and a protein involved in promoting cell cycle progression. In addition, Caveolin-1 has an amino acid sequence of SEQ ID No.:1.

According to one embodiment, Caveolin-1 exhibits the same pattern of increase in expression as that of proteins used as an existing biomarker in a cancer cell. For example, a cancer stem cell or a circulating cancer cell that has undergone epithelial-mesenchymal transition has a higher expression level than a normal cell and/or a normal cancer cell, and thus, Caveolin-1 may be used as a biomarker for the diagnosis of cancer, in particular, for detecting a cancer stem cell or a circulating cancer cell that has undergone epithelial-mesenchymal transition.

According to another embodiment of the present invention, there is provided a kit for detecting a cancer stem cell or a circulating cancer cell that has undergone epithelial-mesenchymal transition, the kit including an antibody specifically binding to Caveolin-1 or a fragment thereof or an antigen binding fragment thereof.

The fragment of Caveolin-1 may be, for example, an immunogenic fragment, and indicates a fragment of a biomarker protein having at least one epitope that is recognizable by an antibody against Caveolin-1, which is a biomarker protein.

A kit for the diagnosis of cancer according to an embodiment of the present invention may be manufactured using one of the methods known in the art, and may typically include freeze-dried antibody and buffer, a stabilizer, inactive protein, and the like. The antibody may be labeled with radionuclides, fluorescors, or enzyme.

According to one embodiment, the kit may be used to detect a cancer cell, for example, a cancer stem cell or a circulating cancer cell that has undergone epithelial-mesenchymal transition without being limited thereto.

The antibody may be a polyclonal antibody or a monoclonal antibody. The polyclonal antibody may be produced by injecting a biomarker protein or a fragment thereof, as an immunogen, to a foreign host, according to one of various methods known in the art. The foreign host includes mammals such as a mouse, a rat, a sheep, and a rabbit. The immunogen may be injected through intramuscular, intraperitoneal or subcutaneous injection, and in this case, may be generally administered together with adjuvant for improving antigenicity. Afterwards, blood is periodically collected from the foreign host to collect blood serum showing improved titer and antigenic specificity, from which an antibody is separated and purified.

The monoclonal antibody may be produced by a technology for producing immortalized cell lines by fusion known in the art. Hereinafter, the production method will be simply described. First, an appropriate amount (approximately 10 µg) of pure protein is obtained, and is immunized into Balb/C mouse. Otherwise, a polypeptide fragment of the pure protein is synthesized, bound to bovine serum albumin, and immunized into a mouse. Then, antigen-producing lymphocyte separated from the mouse is fused with myeloma of a human or a mouse to produce immortalized hybridoma. Then, ELISA method is used to select and proliferate only a hybridoma cell producing a desired monoclonal antibody, and the monoclonal antibody may be separated and purified from the culture.

The monoclonal antibody may be variously used for an immunoassay kit (e.g., ELISA, antibody coated tube test, lateral-flow test, potable biosensor), and may be also used to develop a protein chip having a detection spectrum for various cancer cells through development of an antibody showing higher specificity and sensitivity.

In an embodiment, the antigen binding fragment may be selected from the group consisting of an scFv fragment, a (scFv)₂ fragment, a Fab fragment, a Fab' fragment, and a F(ab')₂ fragment, but is not limited thereto. The term "antigen binding fragment" used herein refers to fragments of an intact immunoglobulin, and any part of a polypeptide including antigen binding regions. A Fab fragment has one antigen binding site and contains the variable regions of a light chain and a heavy chain, the constant region of the light chain, and the first constant region C_{H1} of the heavy chain. A Fab' fragment is different from the Fab fragment in that the Fab' fragment additionally includes the hinge region of the heavy chain, including at least one cysteine residue at the C-terminus of the heavy chain C_{H1} region. A F(ab')₂ fragment is produced whereby cysteine residues of the Fab' fragment are joined by a disulfide bond at the hinge region. An Fv fragment is the minimal antibody fragment having only heavy chain variable regions and light chain variable regions, and a recombinant technique for producing the Fv fragment is well known in the art. Two-chain Fv fragments may have a structure in which heavy chain variable regions are linked to light chain variable regions by a noncovalent bond. Single-chain Fv fragments generally may have a dimer structure as in the two-chain Fv fragments in which heavy chain variable regions are covalently bound to light chain variable regions via a peptide linker or heavy and light chain variable regions are directly linked to each other at the C-terminus thereof. The antigen binding fragment may be obtained using a protease (for example, a whole antibody is digested with papain to obtain Fab fragments, or is digested with pepsin to obtain F(ab')₂ fragments), and may be prepared by a genetic recombinant technique.

According to another embodiment of the present invention, there is provided a method of detecting Caveolin-1 in a biological sample, in particular in order to provide information needed for diagnosis of cancer, the method including detecting, from a biological sample likely to have cancer, a cancer stem cell or a circulating cancer cell that has undergone epithelial-mesenchymal transition, and which thus has a higher expression level of Caveolin-1 than a normal cell; and determining from the detection results whether cancer is developed in the biological sample.

According to the method of detecting Caveolin-1 from a biological sample, the method may include detecting from a biological sample likely to have cancer a cancer stem cell or a circulating cancer cell that has undergone epithelial-mesenchymal transition, and which thus has a relatively higher expression level of Caveolin-1 than a normal cell.

The detecting process may be performed using the kit for detecting a cancer stem cell or a circulating cancer cell that has undergone epithelial-mesenchymal transition, and may be performed by immunoassay. Immunoassay may be prepared according to immunoassay or immunostaining protocols which have been conventionally developed. Examples of the immunoassay or immunostaining methods include radioimmunoassay, radioimmunoprecipitation, immunoprecipitation, enzyme-linked immunosorbent assay (ELISA), capture-ELISA, inhibition or competition assay, sandwich analysis, flow cytometry, immunofluorescence staining, and immunoaffinity purification, but are not limited thereto. For example, in an embodiment of a radioimmunoassay method, a radioisotope-labeled antibody may be used to detect Caveolin-1. The radioisotope may be, for example, C¹⁴, I¹²⁵, P³² or S³⁵.

In an embodiment of an ELISA method, the method may include: (i) coating a surface of a solid substrate with a blood sample of each of a normal person and a subject likely to have cancer; (ii) contacting the blood sample with an antibody specifically binding to Caveolin-1 or a fragment thereof as a primary antibody to induce an antigen-antibody reaction; (iii) reacting the resultant product obtained by (II) process with a secondary antibody with an enzyme conjugated thereto; and (iv) detecting the activity of the enzyme.

The solid substrate may be a hydrocarbon polymer such as polystyrene or polypropylene, glass, a metal, or a gel. For example, the solid substrate may be a microtiter plate. The enzyme conjugated to a secondary antibody may be an enzyme catalyzing a colorimetric, fluorometric, luminescence or infra-red reactions, but is not limited thereto. For example, the enzyme may be alkaline phosphatase, β-galactosidase, horseradish peroxidase, luciferase, or Cytochrome P₄₅₀. When alkaline phosphatase is used, bromo-chloro-indolyl-phosphate (BCIP), nitro blue tetrazolium (NBT), naphthol-AS-B1-phosphate, or enhanced chemifluorescence (ECF) may be used as a substrate. When horseradish peroxidase is used, chloronaphtol, aminoethylcarbazol, diaminobenzidine, D-luciferin, lucigenin (bis-N-methylacridinium nitrate), resorufin benzyl ether, luminol, Amplex Red reagent (10-acetyl-3,7-dihydroxyphenoxazine), hypersensitive reaction solution (HYR: p-phenylenediamine-HCl and pyrocatechol), tetramethylbenzidine (TMB), 2,2'-Azine-di[3-ethylbenzthiazoline sulfonate] (ABTS), o-phenylenediamine (OPD) and naphtol/pyronin, glucose oxidase and t-nitroblue tetrazolium (t-NBT), or m-phenzaine methosulfate (m-PMS) may be used as a substrate.

In an embodiment of a capture-ELISA method, the method may include: (i) coating a surface of a solid substrate with an antibody specifically binding to Caveolin-1 or a fragment thereof as a capturing antibody; (ii) contacting the capturing antibody and a blood sample of a subject likely to have cancer to induce an antigen-antibody reaction; (iii) reacting the resultant product obtained by (II) process with a detecting antibody with a signal-generating label attached thereto and specifically binding to Caveolin-1; and (iv) detecting the signal generated from the label. The detecting antibody may have a label generating a detectable signal. The label may be a chemical label such as biotin; an enzymatic label such as alkaline phosphatase, β-galactosidase, horseradish peroxidase and Cytochrome P₄₅₀; a radioactive label such as C¹⁴, I¹²⁵, P³² and S³⁵; a fluorescent label such as fluorescein; a luminescent label; chemiluminescent label; or a fluorescence resonance energy transfer (FRET) label, but is not limited thereto.

The final measurement of enzyme activities or signals in the ELISA method and the capture-ELISA method may be performed by any method known to one skilled in the art to enable quantitative or qualitative analysis of Gaveolin-1. For example, signals may be detected easily by streptavidin in the case of a biotin-labeled antibody and by luciferin in the case of a luciferase-labeled antibody.

As the kit described above, a microchip or an automated microarray system that can detect an antigen for the antibody by immobilizing an antibody specifically binding to Caveolin-1 or a fragment thereof on a microchip and then reacting the antibody with a biological sample isolated from a subject. By using this, a large amount of a biological sample can be analyzed at once.

According to one embodiment, the biological sample may be any sample containing a cancer stem cell or a circulating cancer cell that has undergone epithelial-mesenchymal transition. For example, the biological sample may be selected from the group consisting of blood, bone marrow fluid, lymph fluid, saliva, lachrymal fluid, urine, mucous fluid, amniotic fluid, and combinations thereof, but is not limited thereto.

The method of detecting Caveolin-1 may include determining from the detection results whether cancer is developed in the biological sample.

In other words, cancer may be determined/diagnosed by analyzing the intensity of a final signal from the immunoassay described above. For example, when the signal for Caveolin-1 from a sample (e.g., blood) of a subject likely to have cancer is stronger than the corresponding signal from a normal control sample, the subject may be diagnosed/determined as having cancer or at least as having an improved risk of cancer.

According to another embodiment of the present invention, a method of isolating a cancer stem cell or a circulating cancer cell that has undergone epithelial-mesenchymal transition includes detecting from a biological sample a cancer stem cell or a circulating cancer cell that has undergone epithelial-mesenchymal transition, in which Caveolin-1 is expressed on the surface thereof; and isolating from the biological sample the detected cancer stem cell or the detected circulating cancer cell that has undergone epithelial-mesenchymal transition.

According to the method of isolating cancer cells, first, the method may include detecting from a biological sample a cancer stem cell or a circulating cancer cell that has undergone epithelial-mesenchymal transition, in which Caveolin-1 is expressed on the surface thereof.

According to one embodiment, the cancer cell may be a cancer stem cell or a circulating cancer cell that has undergone epithelial-mesenchymal transition, but is not limited thereto.

As described above, Caveolin-1 may be used as a biomarker for detecting a cancer cell, for example, a cancer stem cell or a circulating cancer cell that has undergone epithelial-mesenchymal transition. Thus, the detection of Caveolin-1 may be performed using an antibody against Caveolin-1 or an antigen binding fragment thereof. For example, the detecting process may be performed using an antibody against Caveolin-1 or an antigen binding fragment thereof and beads to which a detectable label such as a fluorescent label is bound, through an antigen-antibody reaction with Caveolin-1 on surfaces of cancer cells contained in a biological sample.

After the detecting process, the method may include isolating from the biological sample the detected cancer stem cell or the detected circulating cancer cell that has undergone epithelial-mesenchymal transition.

For example, when an antibody against Caveolin-1 or an antigen binding fragment thereof and beads to which a detectable label such as a fluorescent label is bound are used to detect cancer cells, the isolating process may be performed by centrifugation, filtration, or chromatography. The isolated cancer cells may be cultured using a culturing method well known to one of ordinary skill in the art, thereby being suitable for use in experiments.

One or more embodiments of the present invention will now be described more fully with reference to the following examples. However, these examples are provided only for illustrative purposes and are not intended to limit the scope of the present invention.

### Example 1: Detection of protein expressed in breast cancer cell lines having a low expression level of EpCAM through microarray

51 types of breast cancer cell lines were divided according to whether or not EpCAM was expressed, with reference to Cancer Cell, 10(6):515-527, 2006, and proteins expressed on surfaces of the breast cancer cell lines were analyzed using microarray data. From the microarray data, it was confirmed that 20 breast cancer cell lines including R 75-1, SKBR3, MCF-7, and the like have a high expression level of EpCAM, and it was confirmed that 31 breast cancer cell lines including MDA231, MDA436, MCF10A, and the like have a low expression level of EpCAM. As a result of analysis, it was confirmed that the breast cancer cell lines with a low expression level of EpCAM had a high expression level of Caveolin-1.

### Example 2: Confirmation of expression level of Caveolin-1 according to cancer cell lines

An expression level of Caveolin-1 in various kinds of cancer cell lines was confirmed by western blotting. 9 types of cancer cell lines (purchased from (ATCC (American Type Culture Collection)) including breast cancer cell lines (i.e., ZR75-1, SKBR3, MCF7, MDA-MB-231, MDA-MB-436, MCF10A) and prostate cancer cell lines (i.e., PC3, LnCAP, DU145) were cultured in a DMEM medium in a 100 mm culture dish, and then cell extracts were obtained therefrom. 20 µg of each cell extract was isolated using a Novex NuPAGE Bis-Tris Electrophoresis System (Invitrogen) and then transferred on a Nitrocellulose membrane (Invitrogen, cat.no #LC2006). Each membrane was blocked in 3% skim milk for 1 hour and then reacted with Caveolin-1 antibodies (AbCAM, cat.no #2910) diluted to 1:1000 at 4 °C for 18 hours or longer. Then, the resultant membrane was fully washed with a TBS-T solution to remove unreacted antibodies, and each resultant membrane was reacted with goat anti-rabbit IgG-horseradish peroxidase (HRP) at room temperature for 1 hour. Afterwards, the resulting membranes were fully washed with a TBS-T solution, and a peroxidase substrate solution (Thermo Scientific Pierce ECL Western Blotting Substrate, cat.no#32106) was added thereto to generate fluorescence. The generated fluorescence was measured to compare the expression levels of Caveolin-1 in the 9 types of breast cancer cell lines with one another.

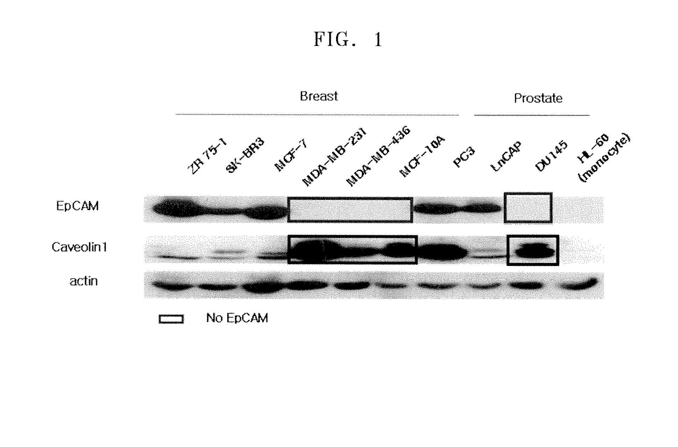

As a result, as shown in FIG. 1, it was confirmed that the MDA-MB-231, MDA-MB-436 and DU145 cell lines that had no expression of EpCAM from among the 9 types of breast cancer cell lines had a strong expression level of Caveolin-1.

### Example 3: Production of beads with Caveolin-1 antibodies bound thereto

COOH polystyrene beads having a diameter of 1 or 3 µm were treated with EDC(N-hydroxysuccinimide)/NHS(1-ethyl-3-[3-dimethylaminopropyl]carbodiimide hydrochloride), the treated beads were added to the prepared PBS solution, 0.65 mg/ml of antibodies specifically binding to Caveolin-1 were added thereto, and the resulting solution was slowly shaken at room temperature for 2 hours, thereby completing the production of beads to which antibodies specifically binding to Caveolin-1 were bound.

### Example 4: Artificially induced epithelial-mesenchymal transition in breast cancer cell lines

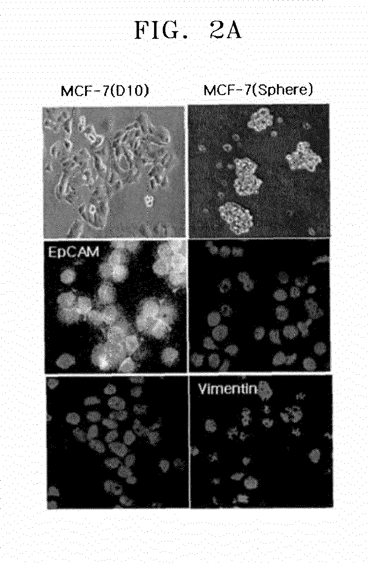

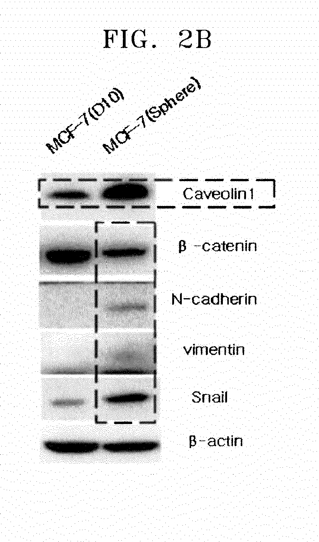

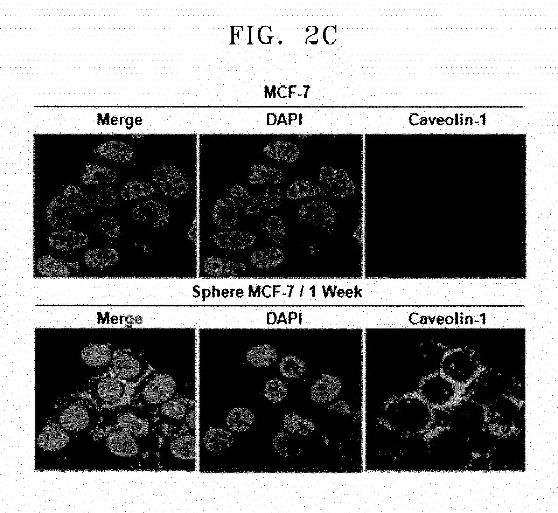

To induce epithelial-mesenchymal transition in breast cancer cell lines MCF7, a mommosphere culture method described below was used instead of the existing attachment culture (DMEM + 10% FBS) method. A medium containing DMEM-F12, 1 x B27, 20 ng/ml FGF, 20 ng/ml EGF, and 5 ug/ml insulin was used as a culture medium, and bacteria cells (2 x 10⁵ cells/ml) were inoculated in a 100 mm dish and then cultured for 1 week. After the culturing process, immunocytochemistry was performed on the cultured bacteria cells. As a result, as shown in FIG. 2A, it was confirmed that there was no expression of EpCAM, which is an epithelial marker, and the expression of vimentin, which is a mesenchymal marker, increased. In addition, as a result of western blotting, as shown in FIG. 2B, it was confirmed that epithelial-mesenchymal transition was induced by confirming that the amount of β-catein, which is a protein known to have decreased expression when epithelial-mesenchymal transition was induced, decreased, and the amounts of snail, N-cadherin, and vimentin, which are proteins known to have increased expression when epithelial-mesenchymal transition was induced, increased. In this regard, as seen in FIGS. 2B and 2C, it was confirmed that the expression level of Caveolin-1 significantly increased when epithelial-mesenchymal transition was induced.

### Example 5: Confirm the binding affinity of beads with a Caveolin-1 antibody bound thereto to circulating cancer cells that had undergone epithelial-mesenchymal transition

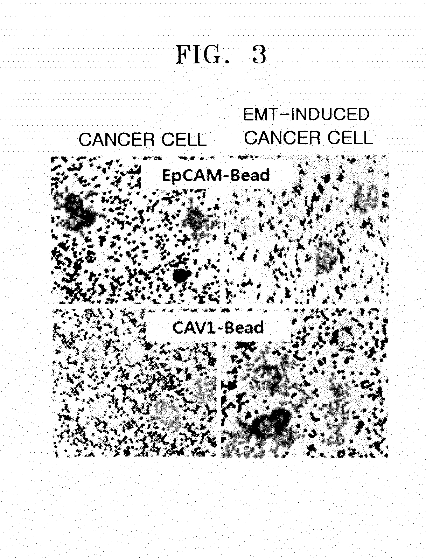

To confirm whether or not Caveolin-1 was expressed on surfaces of MCF-7 cell lines that had undergone epithelial-mesenchymal transition, first, as in Example, 4, epithelial-mesenchymal transition was induced in breast cancer cell lines MCF7 with a high expression level of EpCAM. Subsequently, 30 µl of the beads with a Caveolin-1 antibody bound thereto which were prepared according to Example 3 was added to 1 x 10⁵ MCF-7 cell lines suspended in a DMEM medium and left for 1 hour. Then, whether the beads were bound to the MCF-7 cell lines was confirmed through the fluorescence intensity of fluorescein by using a fluorescence microscope (Olympus IX-81). In this example, beads with an EpCAM antibody bound thereto were used as a control. As a result, as seen in FIG. 3, it was confirmed that the binding of the beads with a Caveolin-1 antibody bound thereto to the MCF-7 cell lines that had undergone epithelial-mesenchymal transition notably increased as compared to normal cancer cells. Then, the beads with Caveolin-1 antibodies bound thereto were separated by centrifugation.

### Example 6: Confirmation of Caveolin-1 as a marker for circulating cancer cells that have undergone epithelial-mesenchymal transition or cancer stem cells

To confirm that Caveolin-1 can be used as a marker for circulating cancer cells that have undergone epithelial-mesenchymal transition or cancer stem cells, western blotting was used to confirm whether or not Snail known as a marker for circulating cancer cells that have undergone epithelial-mesenchymal transition and ALDH1, CD133, and CD44, which are known as markers for cancer stem cells, was expressed in breast cancer cell lines MCF7 in which epithelial-mesenchymal transition was induced. The western blotting process may be performed in the same manner as in Example 2, except that a Caveolin-1 antibody (AbCAM, cat.no #2910), a Snail antibody (Cell signaling cat.no #3879), an ALDH1 antibody (AbCAM, cat.no #23375), a CD133 antibody (AbCAM, cat.no #27699), and a CD44 antibody (Cell signaling cat.no #5640) were respectively used for the markers as a primary antibody.

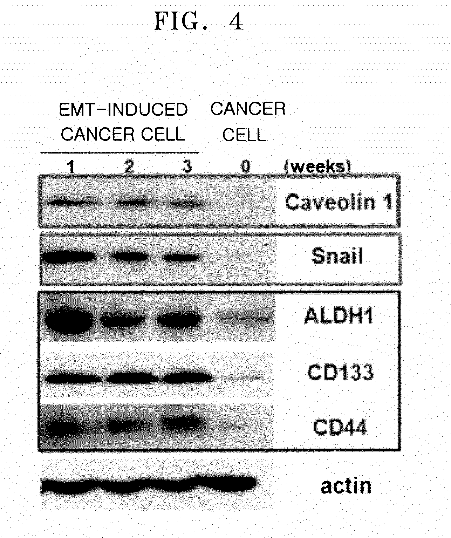

As a result, as seen in FIG. 4, it was confirmed that the expression levels of Caveolin-1, Snail, ALDH1, CD133, and CD44 increased in the breast cancer cell lines MCF7 in which epithelial-mesenchymal transition was induced. In view of the results that Caveolin-1 exhibited the same pattern of increase in expression as that of Snail, ALDH1, CD133, and CD44, this indicates that Caveolin-1 may also be used as a marker for circulating cancer cells that have undergone epithelial-mesenchymal transition or a marker for cancer stem cells.

As described above, according to the one or more of the above embodiments of the present invention, by using a biomarker for cancer diagnosis and a kit including an antibody against the biomarker, a cancer stem cell or a circulating cancer cell that has undergone epithelial-mesenchymal transition may be efficiently detected and isolated.

It should be understood that the exemplary embodiments described therein should be considered in a descriptive sense only and not for purposes of limitation. Descriptions of features or aspects within each embodiment should typically be considered as available for other similar features or aspects in other embodiments.

## Claims

1. A biomarker for detecting a cancer stem cell or a circulating cancer cell that has undergone epithelial-mesenchymal transition, the biomarker comprising Caveolin-1 having a higher expression level in a cancer cell than a normal cell.

2. A kit for detecting a cancer stem cell or a circulating cancer cell that has undergone epithelial-mesenchymal transition, the kit comprising an antibody specifically binding to Caveolin-1 or an immunogenic fragment thereof or an antigen binding fragment thereof.

3. The kit of claim 2, wherein the antigen binding fragment is selected from the group consisting of an scFv fragment, a (scFv)₂ fragment, a Fab fragment, a Fab' fragment, and a F(ab')₂ fragment.

4. The kit of claim 2, wherein the antibody is a monoclonal antibody.

5. A method of detecting Caveolin-1 in a biological sample, the method comprising:
detecting in the biological sample a cancer stem cell or a circulating cancer cell that has undergone epithelial-mesenchymal transition, which has a higher expression level of Caveolin-1 than a normal cell; and
determining from the detection results whether cancer has developed in the biological sample.

6. The method of claim 5, wherein the biological sample is selected from the group consisting of blood, bone marrow fluid, lymph fluid, saliva, lachrymal fluid, urine, mucous fluid, amniotic fluid, and combinations thereof.

7. A method of isolating a cancer stem cell or a circulating cancer cell that has undergone epithelial-mesenchymal transition, the method comprising:
detecting in a biological sample a cancer stem cell or a circulating cancer cell that has undergone epithelial-mesenchymal transition, wherein Caveolin-1 is expressed on a surface of the cancer stem cell or the circulating cancer cell; and
isolating from the biological sample the detected cancer stem cell or the detected circulating cancer cell that has undergone epithelial-mesenchymal transition.

8. The method of claim 7, wherein the biological sample is selected from the group consisting of blood, bone marrow fluid, lymph fluid, saliva, lachrymal fluid, urine, mucous fluid, amniotic fluid, and combinations thereof.

9. The method of any one of claims 5 to 8, wherein the a cancer stem cell or a circulating cancer cell that has undergone epithelial-mesenchymal transition is detected using the kit of any one of claims 2 to 4.


Sequence Listing

<110>    Samsung Electronics Co. Ltd

<120>    Biomarkers for diagnosing cancer and method for isolating
         cancer cell using the same

<130>    EP83746FZTRpau

<140>    not yet assigned
<141>    2012-01-10

<150>    10-2012-0003081
<151>    2012-01-10

<160>    1

<170>    KopatentIn 1.71

<210>    1
<211>    178
<212>    PRT
<213>    Homo sapiens

<400>    1
Met Ser Gly Gly Lys Tyr Val Asp Ser Glu Gly His Leu Tyr Thr Val
  1               5                  10                  15

Pro Ile Arg Glu Gln Gly Asn Ile Tyr Lys Pro Asn Asn Lys Ala Met
             20                  25                  30

Ala Asp Glu Leu Ser Glu Lys Gln Val Tyr Asp Ala His Thr Lys Glu
         35                  40                  45

Ile Asp Leu Val Asn Arg Asp Pro Lys His Leu Asn Asp Asp Val Val
     50                  55                  60

Lys Ile Asp Phe Glu Asp Val Ile Ala Glu Pro Glu Gly Thr His Ser
 65                  70                  75                  80

Phe Asp Gly Ile Trp Lys Ala Ser Phe Thr Thr Phe Thr Val Thr Lys
                 85                  90                  95

Tyr Trp Phe Tyr Arg Leu Leu Ser Ala Leu Phe Gly Ile Pro Met Ala
            100                 105                 110

Leu Ile Trp Gly Ile Tyr Phe Ala Ile Leu Ser Phe Leu His Ile Trp
        115                 120                 125

Ala Val Val Pro Cys Ile Lys Ser Phe Leu Ile Glu Ile Gln Cys Ile
    130                 135                 140

Ser Arg Val Tyr Ser Ile Tyr Val His Thr Val Cys Asp Pro Leu Phe
145                 150                 155                 160

Glu Ala Val Gly Lys Ile Phe Ser Asn Val Arg Ile Asn Leu Gln Lys
                165                 170                 175

Glu Ile